## Europäisches Patentamt
## European Patent Office
## Office européen des brevets

(19)

(11) Veröffentlichungsnummer: **0 121 912**
A1

(12) **EUROPÄISCHE PATENTANMELDUNG**

(21) Anmeldenummer: 84103815.1

(51) Int. Cl.³: **C 07 C 103/52**, G 01 N 33/68

(22) Anmeldetag: 06.04.84

(30) Priorität: 07.04.83 US 482383

(43) Veröffentlichungstag der Anmeldung: 17.10.84 Patentblatt 84/42

(84) Benannte Vertragsstaaten: **CH DE FR GB IT LI NL**

(71) Anmelder: **F. HOFFMANN-LA ROCHE & CO. Aktiengesellschaft, CH-4002 Basel (CH)**

(72) Erfinder: **Morgan, James I., 215 Alexander Avenue, Upper Montclair, N.J. 07043 (US)**

(74) Vertreter: **Freiherr von Pechmann, Eckehart et al, Patentanwälte Wuesthoff- v. Pechmann-Behrens-Goetz Schweigerstrasse 2, D-8000 München 90 (DE)**

(54) Radioimmunverfahren zur Bestimmung von Thymosin-beta-4.

(57) Es wird ein Radioimmunverfahren zur Bestimmung von Thymosin $\beta_4$ beschrieben. Das in der Probe vorhandene Thymosin $\beta_4$ konkurriert mit tritiiertem Thymosin $\beta_4$ um die vorhandenen Bindungsstellen der Thymosin $\beta_4$-Antikörper. Der gebildete Antigen/Antikörper-Komplex wird von nicht gebundenem tritiiertem Thymosin $\beta_4$ abgetrennt, worauf in der Festphase das tritiierte Thymosin $\beta_4$ gemessen und mit einer Standardkurve verglichen wird.

FIG.5A

FIG.5B

EP 0 121 912 A1

F.HOFFMANN-LA ROCHE & CO.Aktiengesellschaft, Basel/Schweiz

0121912

RAN 4093/69

# Radioimmunverfahren zur
## Bestimmung von Thymosin $\beta_4$

Von im Thymus gebildeten hormon-ähnlichen Substanzen hat man lange angenommen, dass sie die Funktion und die Reifung der T-Lymphozyten regulieren. Bis heute wurde eine Anzahl vermeintlicher Thymusfaktoren isoliert, sowie deren biologischen Aktivitäten beschrieben. Vgl. z.B. das U.S. Patent Nr. 4,079,127. Ein solcher Faktor, nämlich das Peptid Thymosin $\beta_4$, wurde aus Thymus isoliert und deren Aminosäuresequenz bestimmt (U.S. Patent Nr. 4,297,276).

Von Thymosin $\beta_4$ wurde berichtet, dass es die Immunantwort modifiziert, was sowohl in in-vivo als auch in in-vitro Methoden bezüglich der Immunfunktion gezeigt wurde. Neue biochemische Studien haben die Gegenwart erheblicher Mengen von Thymosin $\beta_4$ (im folgenden $\beta_4$) in Extrathymusgewebe gezeigt und ebenfalls die spezifische Synthese dieses Peptids durch Kulturen von peritonealen Macrophagen von Ratten gezeigt.

Aus diesem Grunde ist die Verfügbarkeit von für $\beta_4$ spezifischen Antikörpern sowie von radioaktiv markiertem $\beta_4$ für die Verwendung in einem Radioimmunverfahren, bei dem die physiologischen Spiegel dieses Peptides aufgefunden werden können, von grosser Bedeutung. Dies ist besonders

Klt/22.3.84

wichtig im Hinblick darauf, dass gewisse vorläufige Beobachtungen zeigen, dass die Spiegel von im Blut von Patienten mit autoimmunen oder sklerotischen Krankheiten, wie solchen die an demyelisierenden Krankheiten leiden, zirkulierenden $\beta_4$ wesentlich unter den normalen $\beta_4$-Spiegeln liegen. Man nimmt an, dass dies die mögliche Freigabe von $\beta_4$ während dieser destruktiven Phasen dieser Krankheiten zusammen mit der gleichzeitigen Bildung von Autoantikörpern zu diesem Peptid widerspiegelt. Aus diesem Grund kann ein Radioimmunverfahren für $\beta_4$ ein wertvolles Instrument für die Beobachtung früher Stadien dieser Krankheiten sowie auch zur Beobachtung des Fortschreitens sowie des Erfolges der Therapie für diese Krankheiten sein.

Die vorliegende Erfindung betrifft ein Radioimmunverfahren für Thymosin $\beta_4$. Der in diesem Verfahren verwendete Antikörper wird zweckmässigerweise so hergestellt, dass man Thymosin $\beta_4$ unter Bildung eines Immunreagens kovalent an ein herkömmliches immunologisches Trägermaterial bindet. Die Quelle für Thymosin $\beta_4$ ist nicht kritisch für die Zwecke der vorliegenden Erfindung. Geeignetes Thymosin $\beta_4$ kann aus Fraktion 5 von Säugern verschiedener Quelle verwendet werden. So kann z.B. Thymosin $\beta_4$ von Fraktion 5 von Menschen, Rindern, Schafen oder Schweinen verwendet werden. Dies ist möglich dank der Homologie der Aminosäuresequenzen von Thymosin $\beta_4$ von diesen verschiedenen Säugerspezies.

Alternativ und vorzugsweise erhält man Thymosin $\beta_4$ durch Peptidsynthese nach an sich bekannten Methoden. So ist z.B. eine Synthese von Thymosin $\beta_4$ im U.S. Patent Nr. 4.297.276 beschrieben.

Der in dieser Beschreibung verwendete Ausdruck "immunogenes Trägermaterial" bzw. "immunologisches Trägermaterial" umfasst diejenigen Materialien, welche unabhängig in der Lage sind, in einem Wirtstier eine immunogene Antwort her-

0121912

vorzurufen. Weiterhin bedeutet dieser Ausdruck, dass das Material entweder direkt über die Bildung von Peptid- oder Esterbrücken zwischen freien Carboxyl-, Amino- oder Hydroxylgruppen in Thymosin $\beta_4$ und entsprechenden Gruppen im immunogenen Trägermaterial oder alternativ durch konventionelle Bindungen mit bi-funktionellen Bindegruppen an Thymosin $\beta_4$ gekoppelt werden kann.

Das kovalente Koppeln von Thymosin $\beta_4$ an den immunogenen Träger kann in herkömmlicher Art und Weise durchgeführt werden. So z.B. ist es möglich, für das direkte Kuppeln ein Carbodiimid, vorzugsweise Dicyclohexylcarbodiimid oder 1-Aethyl-3-(3-dimethylaminopropyl)carbodiimid als Kupplungsmittel zu verwenden. Bei diesem direkten Kuppeln ist es wünschenswert, ein leicht saures Reaktionsmedium zu verwenden, z.B. ein Medium mit einem pH im Bereich von 3-6,5, besonders bevorzugt im Bereich von 4-6,5.

Ein geeignetes bifunktionelles Bindemittel zur Durchführung der Kopplung ist ein $C_{2-7}$-Dialkanal, wie Glutaraldehyd. Das Koppeln nach dieser Alternativmethode kann bequem nach den Methoden durchgeführt werden, die S. Avrameas, Immunochemistry 6, 43 (1969) beschrieben hat.

Das erhaltene Immunogen kann ohne weitere Reinigung oder alternativ, falls nötig, nach Dialyse zur Entfernung von ungebundenem Thymosin $\beta_4$ und Kupplungsreagenzien verwendet werden.

Geeignete Trägermaterialien, welche bei der Herstellung der Immunogene gemäss vorliegender Erfindung verwendet werden können, umfassen Proteine, natürliche oder synthetische polymere Verbindungen, wie Polypeptide, z.B. Polylysin oder Co-polymere von Aminosäuren, Polysaccharide und dergleichen. Besonders bevorzugte Trägermaterialien sind Proteine und Polypeptide, insbesondere Proteine.

Die Art des bei der Herstellung des Immunogens verwendeten Proteins ist nicht kritisch. Beispiele geeigneter Proteine umfassen Säugerserumproteine, wie z.B. menschliches Gammaglobulin, menschliches Serumalbumin, Rinderserumalbumin, methyliertes Rinderserumalbumin, Kaninchenserumalbumin, Rindergammaglobulin und Pferdegammaglobulin, oder Nicht-Säugerproteine wie z.B. Hämocyanin, besonders Keyhole Limpet Hämocyanin (KLH). Andere geeignete Proteine sind dem Fachmann bekannt.

Die Immunogene gemäss vorliegender Erfindung können zur Bildung von Antikörpern, die spezifisch für $\beta_4$ sind, verwendet werden, indem man sie, vorzugsweise unter Verwendung eines Adjuvans, einem Wirtstier injiziert. Erhöhte Titer können durch wiederholte Injektionen über eine längere Zeitspanne erhalten werden. Geeignete Wirtstiere für diesen Zweck umfassen Säuger, wie Kaninchen, Pferde, Ziegen, Meerschweinchen, Ratten, Kühe, Schafe und dergleichen. Die resultierenden Antiseren enthalten Antikörper, welche selektiv mit Thymosin $\beta_4$ komplexieren. Der radioaktive Ligand der im Radioimmunverfahren gemäss vorliegender Erfindung verwendet wird, ist tritiiertes Thymosin $\beta_4$. Eine solche Tritiierung kann nach bekannten Methoden erfolgen. Eine bevorzugte Methode beinhaltet die Tritiierung durch reduktive Alkylierung der Lysinreste in Thymosin $\beta_4$ in Gegenwart von Formaldehyd unter Verwendung der Methode von Margolis in Analytical Biochemistry 50, 602 (1972). Das erhaltene tritiierte $\beta_4$ wird dann vorzugsweise wieder gereinigt unter Verwendung von Hochdruckflüssigchromatographietechniken (HPLC).

Verschiedene Nachweismethoden können gemäss vorliegender Erfindung verwendet werden. In einem dieser Verfahren werden bekannte Mengen einer zu untersuchenden Probe, Thymosin $\beta_4$ spezifische Antikörper und markiertes Thymosin $\beta_4$ gemischt und stehengelassen. Der Antigen-Antikörperkomplex wird von ungebundenen Reagenzien nach herkömmlichen Techniken getrennt, z.B. durch Behandeln mit Ammoniumsulfat, Polyäthylenglykol, zweitem Antikörper im Ueberschuss oder gebunden an einen unlöslichen Träger, dextranüberzogener Aktivkohle und dergleichen. Die Konzentration von markiertem Thymosin wird entweder in der gebundenen oder ungebundenen Phase bestimmt und der Gehalt an Thymosin $\beta_4$ in der Probe wird dann bestimmt durch Vergleich des Spiegels an markierter Komponentemit einer in an sich bekannter Weise hergestellten Standardkurve. Eine geeignete Standardkurve kann durch Mischen bekannter Mengen von Thymosin $\beta_4$ mit festen Mengen von markiertem $\beta_4$ und Thymosin $\beta_4$ spezifischem Antikörper und Bestimmung des Grades der Bindung für jede dieser bekannten Menge erhalten werden.

Die vorliegende Erfindung wird durch die nachfolgenden Beispiele illustriert.

## Beispiel 1

Herstellung des Immunogens: Ein $\beta_4$-KLH-Konjugat wird durch Glutaraldehyd-Kreuzbindung synthetisiert. 2 mg $\beta_4$ und 18 mg KLH werden in 2 ml 0,1M Phosphatpuffer vom pH 7,2 bei Raumtemperatur gelöst. Die Inkubationsmischung enthält weiterhin 50'000 cpm $\beta_4$, welches biosynthetisch mit [$^{35}$S]-Methionin markiert wurde und durch HPLC wie bei Xu et al. in P.N.A.S. (USA) 79, 4006 (1982) gereinigt wird. Zum Inkubationsgemisch wird dann Glutaraldehyd gegeben bis eine Volumenendkonzentration von 0,6% erhalten wird. Das Ausmass der Konjugation wird durch Entfernen aliquoter Mengen vom Inkubationsgemisch und Trennen der Radioaktivität, die begleitet ist mit $\beta_4$ von höher molekularen Produkten an einer Sephadex G100-Kolonne überwacht. 3 Stunden bei Raumtemperatur waren genügend, um einen signifikanten Spiegel an Konjugation (ungefähr 25%) ohne Bildung einer unkontrollierten Menge von Proteinniederschlag zu erhalten. Nach der Inkubation wird das Gemisch in einen Dialyseschlauch gegeben und über Nacht bei 4°C gegen zwei Wechsel von 0,5 l phosphatgepufferter Kochsalzlösung (PBS) dialysiert. Das dialysierte Produkt wird dann nach Emulsifikation mit Freund's Adjuvans (Gibco) als Immunogen verwendet.

Immunisierung: Männliche weisse Neuseeland-Ratten (Hare) erhielten eine primäre Immunisierung durch wiederholte (30-40) intradermale Injektionen mit 1 mg Konjugat in Freund's vollständigem Adjuvans. Die Tiere werden 5 Wochen später in der gleichen Weise mit 0,8 mg Konjugat in Freund's unvollständigem Adjuvans geboostet. Weitere Boosterinjektionen werden gemäss nachfolgendem Protokol gegeben, welches durch ein Abfallen im Antikörpertiter diktiert wurde. Den Tieren wird Blut routinemässig von der Ohevene entnommen.

Herstellung des radioaktiven Liganden: Thymosin $\beta_4$ (300 μg) wird durch reduktive Alkylierung der Lysinreste in Gegenwart von Formaldehyd und insgesamt 25 mCi Natrium Bor[$^3$H]hydrid (Amersham-Searle 16 Ci/mMol), nach der methode von Margolis (loc. cit.) tritiiert. Das radioaktive Peptid wird wie oben erwähnt durch HPLC wieder gereinigt. Fraktionen der HPLC-Kolonne, welche Radioaktivität enthielten und welche spezifisch mit Antiserum nicht aber mit Nichtimmunserum gefällt werden konnten, werden gesammelt und als Ligand im oben erwähnten RIA verwendet. Die spezifische Aktivität des selektiv gesammelten Liganden war ungefähr 0,7 Ci/mMol. Das radioaktive Produkt wird im folgenden als tritiiertes $\beta_4$ bezeichnet.

<u>Radioimmunverfahren:</u> Tritiiertes $\beta_4$ (7'500 cpm) in 20 µl PBS werden in ein Eppendorf Mikrozentrifugenröhrchen auf Eis gegeben. Hierzu wird Probe oder Standard und 20 µl Antiserum gegeben. Das endgültige Volumen wird mit eiskaltem PBS auf 500 µl eingestellt. Nach 18-stündiger Inkubation bei 4°C wird der Immunkomplex durch die Zugabe von 500 µl gesättigter Natriumsulfatlösung zu jedem Röhrchen gefällt. Nach 1 Stunde werden die Röhrchen bei 15'600 x g während 3 Minuten zentrifugiert, und die Ueberstände werden abgesaugt. Der Niederschlag wird während 2 Stunden in 300 µl Ameisensäure solubilisiert und dann quantitativ in ein Scintillationsröhrchen zum Zählen überführt. Das Radioimmunverfahren wurde durch den Einfluss von gereinigten $\beta_4$-Standards im Bereich von 5-350 pMol quantitativ gemacht.

<u>Herstellung der Proben für den RIA:</u> Die Tiere werden mit $CO_2$ erstickt und die Gewebe entfernt und sofort in flüssigem Stickstoff gefroren. Wenn für das Verfahren benötigt, werden die Gewebe gewogen, in IO ml eiskalter PBS eingetaucht und mit einem Polytron Homogenisator (Brinkmann) getrennt. Nach Zentrifugation bei 30000 x g während 30 Minuten bei 4°C werden aliquote Anteile der Ueberstände direkt auf ihren immunreaktiven Anteil von $\beta_4$ untersucht. In einigen Fällen wird der Ueberstand durch zwei nachfolgende Sep-Pek C18 Patronen (Waters Associates), welche mit PBS äquilibriert sind, geleitet. Nach dem Waschen der Patronen mit PBS wird das $\beta_4$ mit einer Lösung enthaltend 30% Propanol in 0,2M Pyridin, 1,0M Ameisensäure vom pH 3,0 eluiert, wie bei Hannappel et al. in Isolation of peptides From Calf Thymus, Biochem. Biophys. Res. Comm 104, 266 (1982) beschrieben. Die Probe wird dann in zwei aliquote Anteile getrennt und jeder wird in einem Savant Speed Vac Konzentrator getrocknet. Der immunreaktive $\beta_4$-Anteil wird bei einer Probe bestimmt, während die zweite Probe einer HPLC unterworfen und der Anteil an $\beta_4$ im Aliquot durch Aminosäureanalyse bestimmt wird. Zur Abschätzung der Integrität des radioaktiven Liganden unter den Bedingungen des RIA werden rohes Gewebeextrakt und Sep-Pak-gereinigtes Material mit tritiiertem $\beta_4$ inkubiert, worauf das radioaktive Material anschliessend durch HPLC analysiert wird.

HPLC der Peptide: Für die Hochdruckflüssigchromatographie werden Homogenate durch Passieren durch Sep-Pak C18 Patronen in gleicher Weise hergestellt wie die Proben für den RIA. Hochdruckflüssigchromatographietrennung wurde durchgeführt an einer Altex Ultraspher ODS 5 μ (0,45 x 25 cm) Kolonne. Die Peptide werden mit einem linearen Gradienten von 1-Propanol (0-40% Propanol während 210 Minuten bei 0,6 ml/Min.) in 0,2M Pyridin, 1M Ameisensäure eluiert, mit Ausnahme der Produkte der proteolytischen Digestion, bei welchen Acetonitril anstelle von Propanol verwendet wurde. Die Peptidbestimmung wurde nach Reaktion mit Fluorescamin durch Flureszenz bestimmt, wie dies bei Stein und Moschera (1981) High-Performance Liquid Chromatography and Picomole-Level Detection of peptides and Proteins, Methods Enzymol. 79:7 beschrieben wird.

Aminosäurenanalysen: Aminosäurenanalysen wurden bei Anteilen von mit 5,7M HCl bei 15°C und 1 Stunde durchgeführt, wobei ein modifizierter Glenco MM-70 Analysator der für die Verwendung von o-Phthalaldehyd und Fluoreszenz-Bestimmung adaptiert wurde, verwendet wurde.

Proteolytische Digestion: Proben werden in 0,1M $NH_4HCO_3$, 2 mM EDTA vom pH 7,8 während 15 Stunden bei 25°C mit S. aureus V8 digeriert. Digestion mit TPCK-Trypsin wird in 0,4M Pyridin vom pH 7,5 während 15 Stunden bei 25°C durchgeführt.

Herstellung von Antiserum: Ein kovalentes Konjugat von $\beta_4$ und KLH wird wie vorgängig beschrieben durch Glutaraldehyd Kreuzbindung synthetisiert. Alle mit diesem Antigen immunisierten Tiere entwickeln Antikörpertiter zu tritiiertem $\beta_4$ Ligand, wie er als Bindungsspezies für diese Studie verwendet wird. Ein Antiserum, welches als RI bezeichnet wird, wird dann wegen seines hohen Titers ausgefällt und für alle weitern Experimente verwendet.

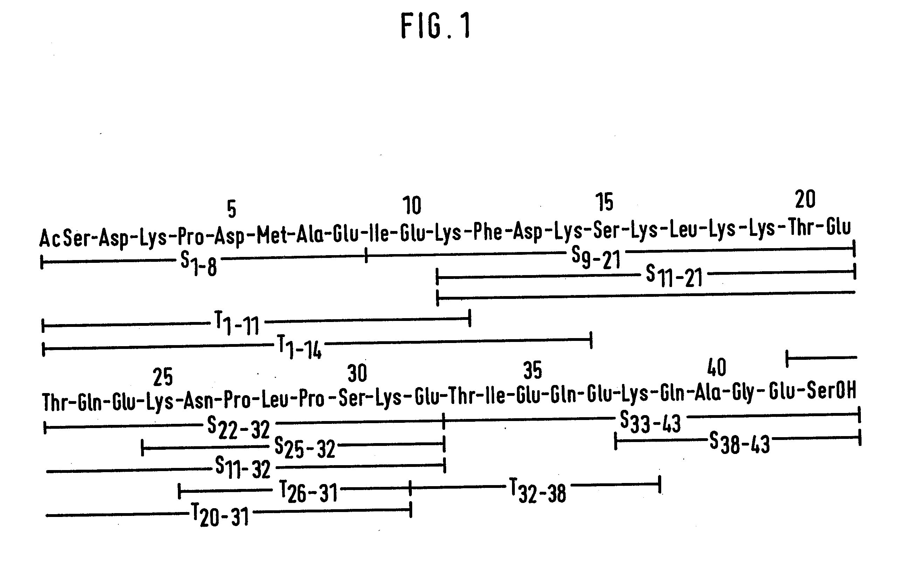

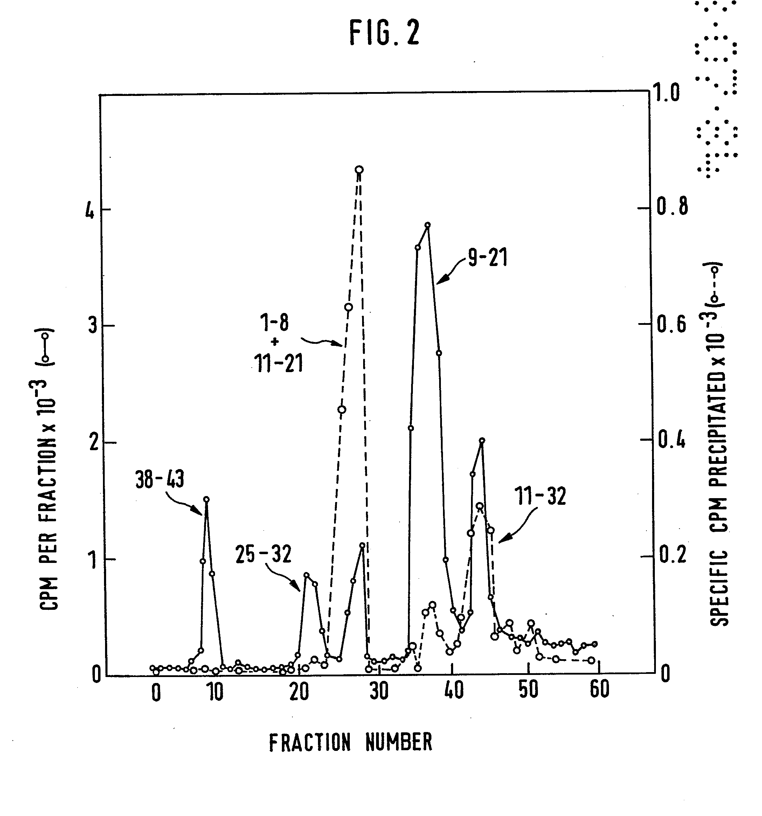

Charakterisierung des Antiserums: Zwei Verfahren werden zur Bestimmung der Epitopspezifität des RI Antiserums verwendet. Im ersten wird tritiiertes $\beta_4$ mit Protease aus S. aureus V8 digeriert und liefert charakteristische Peptide (vgl. Fig. 1), welche durch HPLC isoliert (Fig. 2) und durch Aminosäureanalyse identifiziert werden. Die radioaktiven Fragmente von tritiiertem $\beta_4$ werden dann entweder mit Immun- oder Prä-Immunserum inkubiert und die Antigenantikörperkomplexe gefällt und die Radioaktivität wie früher beschrieben gemessen. Das RI-Antiserum fällt spezifisch Counts von zwei Regionen des HPLC Profils (Fig. 2). Diese Regionen entsprechen dem Peptid umfassend die Reste 11 bis 32 und einem Gemisch von zwei Fragmenten von $\beta_4$, welche die Aminosäuren 1-8 und 11-21 (Fig. 1 und 2) repräsentieren. Da der Hauptpeak der Immunreaktivität in einer heterogenen Region des Chromatogramms beobachtet wurde und da nach diesem Verfahren nur radioaktive ( d.h. Lysin enthaltende) Fragmente studiert werden können, wurde ein weiterer Weg beschritten, um die antigenischen

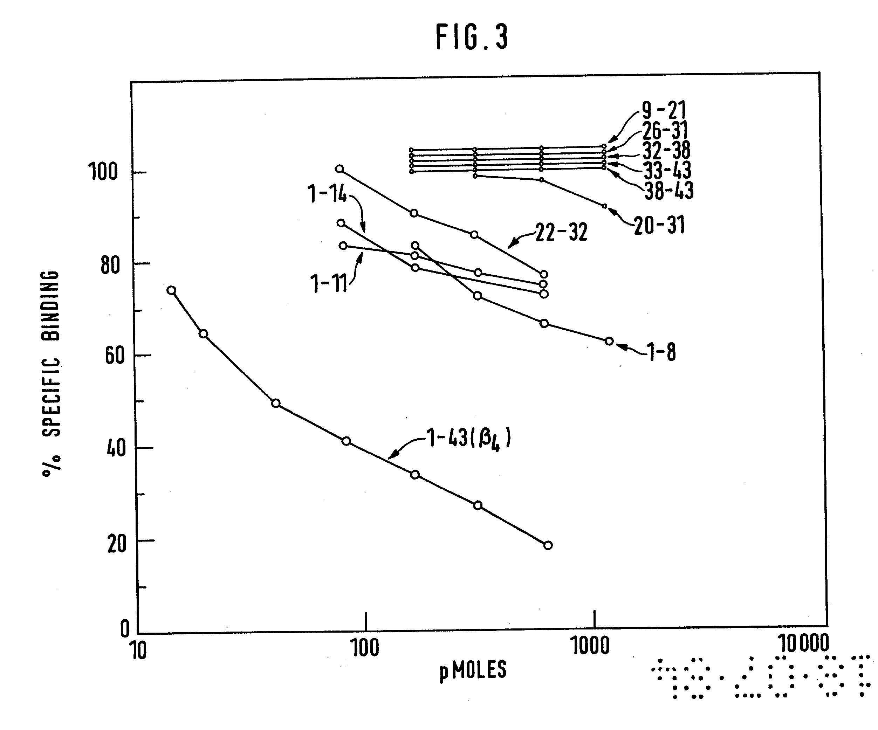

Determinanten des $\beta_4$ Moleküls zu ergründen. Bei diesem Weg wurde das Binden von intakten tritiiertem $\beta_4$ in Gegenwart verschiedener kompetitiver Konzentrationen von Peptidfragmenten abgeleitet von nicht-radioaktivem $\beta_4$ (Fig. 3) quantitativ abgeschätzt. Das Verfahren zur Herstellung dieser Peptide wurde früher beschrieben, und alle Sequenzen sind in Figur 1 dargestellt. Es wurde festgestellt, dass die Aminoterminus-Peptide 1-8, 1-11 und 1-14 im gleichen Ausmass mit dem $\beta_4$ Antiserum kreuzreagieren (Fig. 3). Man benötigt ungefähr 350 pMol dieser N-terminalen Peptide, um eine 25%ige Verdrängung des tritiiertem $\beta_4$ Liganden zu erhalten, wogegen lediglich 15 pMol von authentischem $\beta_4$ erforderlich waren, um ein ähnliches Verdrängen zu erreichen (Fig. 3). Ein anderes Fragment, die Reste 22-32, zeigen ebenfalls eine signifikante Kreuzreaktion, obwohl eine 25%ige Verdrängung des Liganden ungefähr 650 pMol Peptid erfordert (Fig. 3). Das Peptid enthaltend die Reste 20-31 gab nur eine schwache Kreuzreaktion, wogegen fünf andere Fragmente, 9-21, 26-31, 32-38, 33-43 und 38-43 keine Kreuzreaktion bis zur Grenze von 1,2 nMol pro getesteten Versuch ergaben.

Um die Spezifität des Antiserums weiter zu untersuchen wurden mehrere Peptide bezüglich ihrer Eigenschaft tritiiertes $\beta_4$ vom Binden an das RI Antiserum zu verdrängen beobachtet. Eine Anzahl von Peptiden und Proteinen einschliesslich des vermeintlichen Thymushormon $\alpha_1$ wurden getestet und ergaben keine Kreuzreaktion mit dem $\beta_4$ Antiserum bis zu den in Tabelle 1 angegebenen Konzentrationen.

Das im $\beta_4$ Molekül vorhandene Methionin kann leicht mit Wasserstoffperoxyd oxidiert werden. Weiterhin enthalten Extrakte von Zellen, welche $\beta_4$ synthetisieren, zusätzlich zum normalen reduzierten Molekül das Methionylsulphoxyd von $\beta_4$. Es ist deshalb wichtig zu wissen, dass das RI Antiserum nicht unterscheidet zwischen der nativen oder oxidierten Form von $\beta_4$ (Tabelle 1).

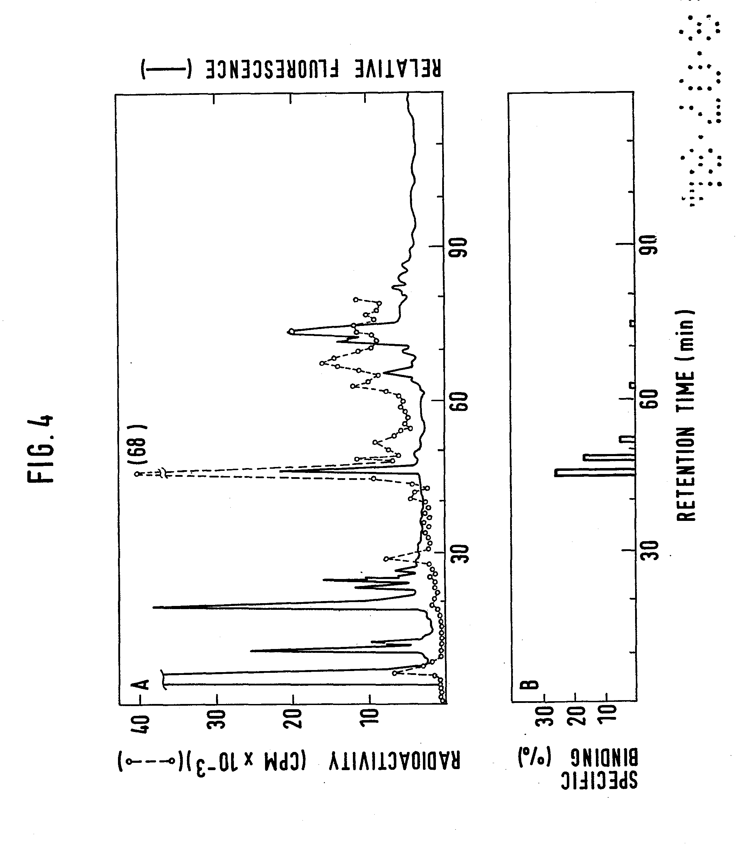

<u>Fällung von [$^{35}$S-]Methionon markiertem $\beta_4$ von Zell-</u><br>
<u>extrakten</u>: Als ein weiterer Test der Spezifität des Antiserums und, fast wichtiger, dessen Brauchbarkeit als ein Werkzeug zum Studium der Biosynthese von $\beta_4$ wurden Experimente durchgeführt, um abzuschätzen, ob die Antikörper signifikante Mengen von radioaktiv markiertem $\beta_4$ von Makrophagenextrakt fällen. Wie in Figur 4 gezeigt, fällt das Antiserum in der Tat Radioaktivität von Regionen des Hochdruckflüssigchromatogramms, welche den Eluations-positionen von $\beta_4$ entsprechen. Wenn unter identischen Bedingungen getestet, fällt das Antiserum keine spezifischen Counts in andern Regionen des Chromatogramms. In andern Experimenten wurde das Fällen von oxidiertem $\beta_4$ beobach-tet. Weiterhin wurde das Fällen von biosynthetischem mar-kiertem $\beta_4$ aus rohen Makrophagenextrakt vor der Hoch-druckflüssigchromatographie beobachtet.

## Beispiel 2

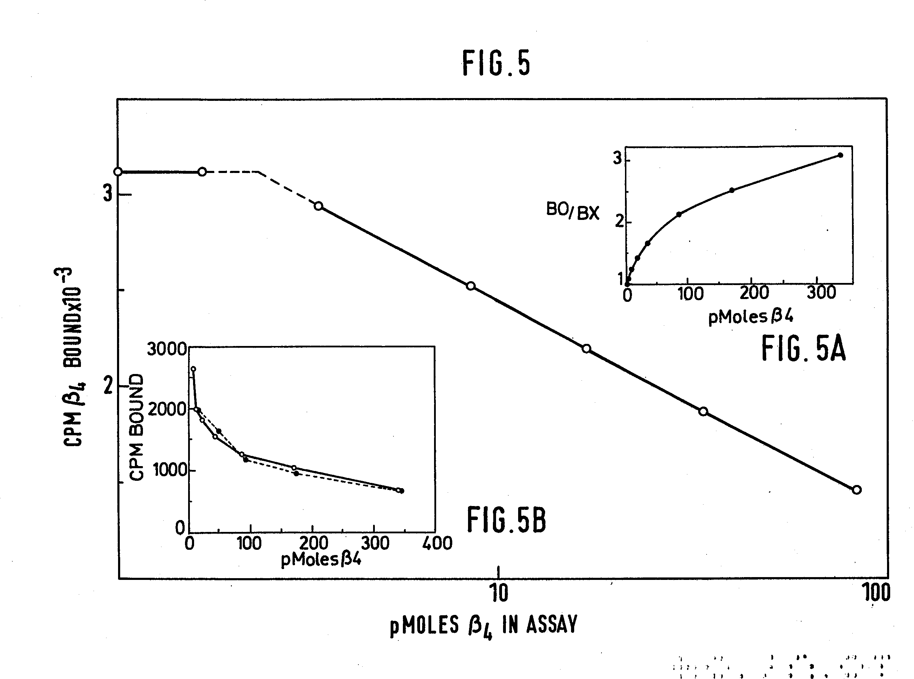

<u>Radioimmunverfahren für $\beta_4$</u>: Ein Radioimmunverfahren wurde unter Verwendung von HPLC gereinigtem tritiiertem $\beta_4$ als Bindungsligand durchgeführt. Details der Verfahrensbedingungen sowie der Synthese und Wiederreinigung von tritiiertem $\beta_4$ sind in Beispiel 1 gegeben. Das Radioimmunverfahren war linear im Bereich von 5-100 pMol $\beta_4$ pro Versuch mit einer unteren Auffindungsgrenze von ungefähr 5 pMol (Fig. 5). Eine Bo/<u>Bx</u>-Aufzeichnung für den $\beta_4$ RIA wird zum Vergleich ebenfalls gegeben (Fig. 5A). Der Auffindungsbereich ist völlig adäquat für die Bestimmung von $\beta_4$ im Rattengewebe (siehe unten); eine Jodierung des Liganden war nicht notwendig. Unter den Verfahrensbedingungen ergibt sich kein bemerkenswerter Abbau des triti-iertem $\beta_4$, noch sind irgendwelche bemerkenswerte quantitative Unterschied im Verhalten von verschiedenen Geweben (Hirn, Milz, Thymus und Lunge) bezüglich der Spaltung von tritiiertem Ligand. Als zusätzliche Kontrolle wurde ein roher Hirnextrakt serienmässig verdünnt, und die $\beta_4$ Mengen wurden durch das Radioimmunverfahren bestimmt (Fig. 5B). Man kann sehen, dass die Werte für das hirnimmunreaktive $\beta_4$ einer Standardkurve für authentisches $\beta_4$ (Fig. 5B) überlagert werden können. Dies würde bedeuten, dass keine offensichtliche Störung mit dem Test von Produkten anders als $\beta_4$, welches im rohen Hirnextrakt vorhanden ist, besteht. Weiterhin wurden alle Gewebeproben bei drei serienmässigen Verdünnungen auf Immunreaktivität $\beta_4$ analysiert, wobei die Resultate mit Ausnahme von Pankreas und Eingeweide bei den verschiedenen Verdünnungen parallel zur Steigung der Standardkurve verliefen. Als endgültiger Beweis des Verfahrens wurden Proben gleichzeitig mittels des Radioimmunverfahrens und mittels Hochdruckflüssigchromatographie (Tabelle 2) auf $\beta_4$ Gehalt geprüft. Wie aus Tabelle 2 hervorgeht, ist eine extrem gute Uebereinstimmung zwischen den Werten vorhanden, die mit den unterschiedlichen Methoden ermittelt wurden.

Thymosin $\beta_4$-Spiegel in Rattengeweben: Wenn das Radioimmunverfahren auf rohe Extrakte von Rattengeweben angewandt wurde, zeigten alle Probe bemerkenswerte Spiegel von immunrekativen $\beta_4$ (Tabelle 3). Die Anwesenheit von authentischem $\beta_4$ in diesen Geweben wurde auch durch Hochdruckflüssigchromatographie gezeigt (Tabelle 2). Muskelgewebe ist sehr arm im $\beta_4$, wogegen die Milz die reichlichste Quelle für dieses Peptid ist (Tabelle) 3). Viele andere Gewebe und Organe haben vergleichbare $\beta_4$-Mengen, die im Bereich von 40-60 µg $\beta_4$-pro Gramm nasses Gewicht liegen. Lunge, Thymus und olfaktorisches Epithel haben einen höheren $\beta_4$-Anteil, nämlich 203, 214 und 126 µg $\beta_4$ pro Gramm Gewicht (Tabelle 3). Zwei Organe, nämlich Eingeweide und Pankreas, zeigten hohe Werte für immunrekatives $\beta_4$, welche mit früher ermittelten Werten gemäss Hochdruckflüssigchromatographie nicht übereinstimmten (Tabelle 3). Nach Reinigung über Sep-Pak zeigten Eingeweide und Pankreas im Radioimmunverfahren niedrigere Werte und waren im Bereich wie bei den meisten Geweben und Organen beobachtet wurde (Tabelle 3). Es wird deshalb vermutet, dass Eingeweide und Pankreas eine proteolytische Aktivität gegen den tritiierten $\beta_4$-Liganden enthalten, obwohl dieser Punkt formell nicht angesprochen wurde. Wie früher erwähnt, zeigen serienmässige Verdünnungen von Pankreas- und Eingeweideextrakten Kurven für immunreaktives $\beta_4$, welche (a) nicht-lineare Anstiege und (b) Anstiege zeigten, die von der Standardkurve signifikant verschieden waren, was ebenfalls auf eine Beeinflussung mit dem Immunverfahren hindeuten könnte. Deshalb müssen gewisse Reinigungen bei diesen Geweben vorgenommen werden, bevor das Radioimmunverfahren möglich ist.

## Tabelle 1

### Kreuzreaktion verschiedener Peptide und Proteine mit $\beta_4$-Antiserum

| Kompetitive Substanz | Kreuzrektivität (%) |
|---|---|
| Thymosin $\beta_4$ (Rind) | 100% (75 pMol) |
| oxidiertes Thymosin $\beta_4$ (Rind) | 96% (75 pMol) |
| Thymosin $\alpha_1$ (Rind) | 0% (20 nMol) |
| Rinderserumalbumin | 0% (56 nMol) |
| Insulin | 0% (20 nMol) |
| Transferrin | 0% (10 nMol) |
| Adrenocorticotropisches Hormon | 0% (10 nMol) |
| Thyroid stimulierendes Hormon | 0% (20 nMol) |

Tabelle 2

Vergleich der Werte für $\beta_4$ in verschiedenen Gewebeproben erhalten durch Hochdruckflüssigchromatographie (HPLC) und Radioimmunverfahren (RIA)

| Quelle des Gewebeextrakts | nMol $\beta_4$ pro Probe | |
|---|---|---|
| | HPLC | RIA |
| Niere | 2,48 | 3,71 ± 0,24 (4) |
| Leber | 3,05 | 2,29 ± 0,18 (6) |
| Lunge | 15,44 | 12,96 ± 2,60 (4) |
| olfaktorisches Epithel | 1,89 | 2,27 ± 0,21 (4) |
| Knochenmark | 1,29 | 1,41 ± 0,13 (4) |
| Haut | 3,41 | 3,68 ± 0,37 (4) |
| Hirn | 7,10 | 8,10 ± 1,24 (6) |
| Milz | 33,63 | 37,27 ± 4,25 (6) |
| Thymus | 9,12 | 8,26 ± 1,07 (6) |

Tabelle 3

Vergleich der immunreaktiven $\beta_4$-Spiegel in verschiedenen Rattengeweben

| Gewebe | $\beta_4$-Anteil (mg $\beta_4$/g Nassgewicht | |
|---|---|---|
| Nieren | 44 ± 6,7 (4) | |
| Eingeweide | 145 ± 37,6 (6) | 38 ± 8,9 (4)* |
| Leber | 41 ± 4,6 (6) | |
| Lunge | 203 ± 23,6 (6) | |
| olfaktorisches Epithel | 126 ± 21,2 (4) | |
| Knochenmark | 55 ± 7,5 (4) | |
| Hoden | 44 ± 4,3 (6) | |
| Herz | 19 ± 3,1 (4) | |
| Haut | 27 ± 3,4 (4) | |
| Hirn | 64 ± 6,4 (6) | |
| Muskel | 8 ± 1,2 (4) | |
| Pankreas | 100 ± 24,3 (6) | 76 ± 10,1 (4)* |
| Milz | 448 ± 67 (6) | |
| Thymus | 214 ± 35 (6) | |

0121912

## Patentansprüche

1. Tritiiertes Thymosin $\beta_4$.

2. Verfahren zur Bestimmung von Thymosin $\beta_4$ in einer Probe, dadurch gekennzeichnet, dass man die Probe mit einer bekannten Menge von tritiiertem Thymosin $\beta_4$ und einem Antikörper mischt, welcher selektiv mit diesem Thymosin $\beta_4$ komplexiert, den erhaltenen Antikörper-Antigenkomplex vom unkomplexierten tritiierten Thymosin $\beta_4$ entfernt, die Menge des gebundenen tritiierten Thymosin $\beta_4$ in diesem Komplex bestimmt und die Menge von Thymosin $\beta_4$ in dieser Probe durch Vergleich des Bindungsgrades mit einer Standardkurve bestimmt.

3. Verfahren nach Anspruch 2, dadurch gekennzeichnet, dass der Antikörper Epitope des Thymosin $\beta_4$ Moleküls entsprechend den Aminosäuren 1-8 und 22-32 erkennt.

4. Verfahren nach Anspruch 2, dadurch gekennzeichnet, dass die Probe eines oder mehrere Fragmente von Thymosin $\beta_4$ enthaltend zumindest die Aminosäuresequenzen 1-8 umfasst.

5. Verfahren nach Anspruch 2, dadurch gekennzeichnet, dass die Probe eines oder mehrere Fragmente von Thymosin $\beta_4$, enthaltend zumindest die Aminosäurensequenz 22-32, enthält.

***

# FIG.1

AcSer-Asp-Lys-Pro-Asp-Met-Ala-Glu-Ile-Glu-Lys-Phe-Asp-Lys-Ser-Lys-Leu-Lys-Lys-Thr-Glu

$S_{1-8}$  $S_{9-21}$  $S_{11-21}$  $T_{1-11}$  $T_{1-14}$

Thr-Gln-Glu-Lys-Asn-Pro-Leu-Pro-Ser-Lys-Glu-Thr-Ile-Glu-Gln-Glu-Lys-Gln-Ala-Gly-Glu-SerOH

$S_{22-32}$  $S_{25-32}$  $S_{11-32}$  $S_{33-43}$  $S_{38-43}$  $T_{26-31}$  $T_{32-38}$  $T_{20-31}$

0121912

# FIG. 2

FRACTION NUMBER

FIG.3

FIG.4

FIG. 5

FIG. 5A

FIG. 5B

**Europäisches Patentamt**

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 013 930 (GEORGE WASHINGTON UNIVERSITY) * Beispiel 2; Ansprüche * ----- | | C 07 C 103/52 G 01 N 33/68 |
| | | | RECHERCHIERTE SACHGEBIETE (Int. Cl. 3) |
| | | | C 07 C 103/52 |

Der vorliegende Recherchenbericht wurde für alle Patentansprüche erstellt.

| Recherchenort BERLIN | Abschlußdatum der Recherche 07-06-1984 | Prüfer BREW C.H. |
|---|---|---|